# EUROPEAN PATENT SPECIFICATION

(11) **EP 1 073 484 B1**
(45) Date of publication and mention of the grant of the patent: **31.08.2005**
(21) Application number: 99921544.5
(22) Date of filing: 30.04.1999
(51) Int. Cl.: A61L 24/00

(54) **ADHESIVE APPLICATOR WITH POLYMERIZATION AGENTS AND/OR BIOACTIVE MATERIAL**
KLEBSTOFF-AUFTRAGVORRICHTUNG MIT POLYMERISATIONSMITTELN UND/ODER BIOAKTIVEM MATERIAL
APPLICATEUR D'ADHESIFS COMPORTANT DES AGENTS DE POLYMERISATION ET/OU UNE SUBSTANCE BIOACTIVE

(30) Priority: 30.04.1998 US 69979
(43) Date of publication of application: 07.02.2001
(73) Proprietor: Closure Medical Corporation, North Carolina 27616 (US)
(72) Inventor: NARANG, Upvan, Raleigh, NC 27613 (US); NICHOLSON, William, Stuart, Cooper, Raleigh, NC 27603 (US)
(74) Representative: Guerre, Dominique
(86) International application number: PCT/US1999/009373
(87) International publication number: WO 1999/055394

(56) References cited:
- WO-A-95/09659
- WO-A-96/40797
- WO-A-97/31598
- WO-A-99/10020
- US-A- 3 759 264

## Description

### BACKGROUND OF THE INVENTION

### 1. Field of the Invention

This invention relates to applicators for applying biomedical adhesives and sealants, methods of making them, and methods of applying such adhesives and sealants. More particularly, this invention relates to methods of applying a polymerization rate modifier, and/or polymerization initiator to an applicator tip; applicators and applicator tips produced by such methods; and methods of using the applicators in medical, surgical, and other topical applications.

### 2. Description of Related Art

Products in primary use for wound closure are surgical sutures and staples. Sutures are recognized to provide adequate wound support. However, sutures cause additional trauma to the wound site (by reason of the need for the needle and suture to pass through tissue and the need to anesthetize the wound area via needle puncture) and are time-consuming to place, and, at skin level, can cause unattractive wound closure marks. Surgical staples have been developed to speed wound apposition and provide improved cosmetic results. However, surgical staples also impose additional wound trauma and require the use of ancillary and often expensive devices for positioning and applying the staples. Both sutures and staples are especially problematic in pediatric cases where the patient may have a strong fear response and refuse to cooperate with their placement, and in geriatric cases where the skin tissue is weaker and prone to tearing.

As an alternate to surgical sutures and staples, adhesives have been proposed for use in wound closure. Similarly, adhesives have been proposed for use in wound covering and protection in such topical applications as surface lacerations, abrasions, stomatitis, and other open surface wounds. One group of such adhesives is the monomeric forms of α-cyanoacrylates.

Typically, for wound closure, the cyanoacrylate surgical adhesive is applied to one or both surfaces of a wound or incision, including the internal portions of the wound, with any excess adhesive being quickly removed from the bonding surfaces. Subsequently, the edges of the wound are held together until they adhere. For example, see U.S. Patent No. 3,559,652 to Coover, Jr. et al. An additional method of application of the cyanoacrylate surgical adhesive to wounds or incisions involves the formation of a bridge over the wound site. As described in U.S. Patent No. 3,667,472 to Halpern, incised tissues are held together and maintained in fixed relationship until a cyanoacrylate adhesive has been applied over the incision and allowed the necessary time to develop a bond.

A method for application of a topical cyanoacrylate tissue adhesive is disclosed in product literature accompanying Histoacryl,® which is commercially available from B. Braun Melsungen AG of Germany. The manufacturer recommends use of this adhesive only for closure of minor skin wounds and not for internal use. Moreover the manufacturer recommends that the adhesive be used sparingly or in thin films because thick films do not increase the film strength and can lead to necrosis of surrounding tissue due to thermogenic polymerization of the cyanoacrylate adhesive.

Typically, when used in medical applications, cyanoacrylate adhesives are applied in monomeric form to the surfaces to be joined, sealed, or otherwise treated. Typically, *in situ* anionic polymerization of the monomer occurs, giving rise to the desired adhesive bond or covering. In these instances, moisture and/or proteins naturally present in the treated tissues initiate polymerization of the adhesives. However, as is the case with Histoacryl,®polymerization can proceed rapidly, with the generation of high levels of heat, which often damage the tissues at or near the site of application.

In an effort to overcome this type of tissue damage, the tissue can first be dried, for example by sponging, to remove essentially all tissue fluids from the site. In this method, there is essentially no water to initiate polymerization. Therefore, polymerization proceeds relatively slowly, often taking greater than 150 seconds, for example. This system, while effective, does not provide a high level of convenience for the user due to the extended time often required for polymerization.

In view of shortcomings associated with the methods disclosed above, an effort has been made to control the rate at which polymerization occurs such that polymerization will occur rapidly enough to be convenient for the user, but not so rapidly that tissue damage occurs due to the polymerization reaction. To control the rate at which the adhesives polymerize (and to improve the shelf life), additives have been included in the monomer adhesive compositions. For example, cyanoacrylate polymerization inhibitors or stabilizers including Lewis acids, such as sulfur dioxide, nitric oxide, boron trifluoride, and other acidic substances, including hydroquinone monomethyl ether, hydroquinone, nitrohydroquinone, catechol, and hydroquinone monoethyl ether have been used. Such inhibitors are disclosed in, for example, U.S. Patent No. 3,559,652 to Banitt, the subject matter of which is incorporated herein by reference. The addition of these inhibitors and stabilizers inhibits premature polymerization of the monomer and slows down the rate of polymerization once the composition is in contact with the tissue to be treated.

Another method for inhibiting polymerization of monomeric adhesives is disclosed in U.S. Patent No. 4,291,131 to McIntire et al. McIntire et al. disclose a nozzle for use on containers for holding cyanoacrylate adhesives so that the cyanoacrylate compositions do not begin to polymerize on exposure to moisture in the air. The nozzle comprises a moldable material having an organic acid dispersed therein that inhibits the polymerization of cyanoacrylates while in the nozzle. The organic acids are incorporated into the moldable material prior to extrusion forming of the nozzle.

Although it is known to add polymerization inhibitors and stabilizers to cyanoacrylate compositions to increase stability and shelf life of the compositions, the addition of polymerization initiators or accelerators to the cyanoacrylate compositions is not widely performed. As discussed above, polymerization typically occurs *in situ* without the need for an external initiator or accelerator. In the situations where an initiator or accelerator is added to the composition, such as when tissue fluids have been removed from the application site, the initiator or accelerator is not added until immediately prior to application of the adhesive. For example, U.S. Patent No. 4,042,442 to Dombroski et al. discloses the addition of a polymerization initiator (either caffeine or theobromine) to a cyanoacrylate adhesive composition. The caffeine or theobromine is added to the adhesive composition in one of two ways. In the first way, the caffeine or theobromine can be mixed with the cyanoacrylate adhesive composition by stirring just prior to application of the adhesive to the substrates to be joined. In the second way, the caffeine or theobromine is dissolved in a volatile solvent, applied to the surfaces to be joined, the volatile solvent is allowed to evaporate, and then the cyanoacrylate adhesive composition is applied to the surfaces of the substrates to be joined. Both of these methods, while effective, are inconvenient for the user because two separate solutions or two separate applications are required.

In an effort to address this inconvenience and lack of control over the polymerization process, published PCT Application No. WO 96/40797, discloses the incorporation of a polymerization initiator or polymerization rate modifier on an applicator tip. Incorporation of the initiator or the rate modifier into the applicator tip provides convenience because only a single composition is required, and allows a level of control over the polymerization rate that cannot be achieved through reliance on polymerization initiators or rate modifiers naturally present at the wound site (such as water). PCT Application No. WO 97/31598 also describes an applicator tip comprising a polymerization initiator.

The polymerization initiators and/or rate modifiers are incorporated into the applicator tip by spraying, dipping, or brushing the applicator tip with a solvent (also referred to herein as a liquid medium) containing the initiator and/or rate modifier. Low boiling point solvents (such as acetone and ethanol, or mixtures thereof) are used to apply the initiator and/or rate modifier.

The applicator tips disclosed in these published PCT applications effectively and conveniently permit mixing of a cyanoacrylate composition with a polymerization initiator or a polymerization rate modifier during dispensing. The polymerization reaction that ensues, however, can be highly exothermic, and, like other methods currently in use, can cause tissue damage at the site of application due to excessive heat generation during polymerization.

In addition to adding polymerization inhibitors, stabilizers, and initiators to monomeric cyanoacrylate compositions, it is also known to add bioactive materials to these adhesive compositions. Often, these bioactive materials are medicaments which are added to the adhesive compositions to aid in the healing process when the cyanoacrylate adhesives are used to close wounds. For example, U.S. Patent No. 5,684,042 to Greff et al. discloses a cyanoacrylate composition comprising an antimicrobially-effective amount of an iodine-containing antimicrobial agent. The iodine-containing antimicrobial agent is dispersible in the cyanoacrylate composition and does not cause premature polymerization of the cyanoacrylate adhesive (i.e., does not initiate polymerization).

Additionally, U.S. Patents Nos. 5,514,371 and 5,624,669 to Leung, et al. disclose the addition of a therapeutic agent in a cyanoacrylate composition. The cyanoacrylate adhesive forms a matrix for the therapeutic agent, with the therapeutic agent being released *in vivo* over time from the matrix during biodegradation of the polymer.

U.S. Patent No. 4,940,579 to Randen discloses a composition comprising a medicament and a cyanoacrylate adhesive. The composition is used to deliver medicaments to non-mucosal areas of mammal bodies.

U.S. Patent No. 5,254,132 to Barley et al. discloses the use of cyanoacrylate adhesives in conjunction with antibiotics. The antibiotics are added to the cyanoacrylate compositions and stored in a sterile applicator for use in a single-dose application. The composition is maintained in a sealed container to avoid polymerization prior to application; therefore, the antibiotic does not initiate or accelerate polymerization of the adhesive composition.

While all of these methods include combining cyanoacrylate adhesives with bioactive materials, the disclosed methods are inconvenient for applying adhesive compositions because multiple solutions and/or applicators are required in order to mix the initiator and adhesive composition or fail to provide a way of controlling the rate at which polymerization proceeds. Furthermore, the selection of bioactive materials has generally been limited by the desire to avoid interaction between the adhesives and the bioactive materials.

### SUMMARY OF THE INVENTION

It has been discovered that the use of methanol, alone or as a component of a mixture of low boiling point solvents, to apply a material (such as a polymerization and/or cross-linking initiator or rate modifier) to an applicator tip used to dispense monomer-containing adhesive compositions, provides an unexpectedly superior distribution profile of the material on, and within, the applicator tip. The superior distribution profile allows a reduction in polymerization time of the dispensed monomeric adhesive while avoiding tissue damage due to the highly exothermic polymerization reaction. It has also been discovered that bioactive materials, which are polymerization initiators and/or rate modifiers as well, can be applied to such applicator tips, providing improved convenience when treating a tissue with a bioactive material.

The present invention provides a method of applying at least one material to an applicator tip used to dispense liquid compositions. In embodiments, the material may be applied to the applicator tip using a solvent comprising methanol and further comprising another low boiling point solvent, such as a low molecular weight ketone or alcohol, or a mixture thereof. As used herein, low molecular weight ketones and alcohols are those which have three or fewer carbon atoms in their main chain. In preferred embodiments, the solvent consists essentially or entirely of methanol.

In embodiments, the material is applied to an applicator tip such that the material is present on the tip in a gradient or anisotropically (i.e., in a pattern that is not identical in all directions within the tip). Preferably, the material is present on the tip in a gradient, where there is a greater amount of the material at the distal end of the tip (the end where the liquid composition exits the applicator tip during dispensing) as compared to the proximal end (the end where the liquid composition enters the applicator tip during dispensing).

In embodiments, the material is an initiator and/or a rate modifier for polymerization and/or cross-linking of a polymerizable monomer. As used herein, a polymerization initiator is any material that causes a monomer composition applied to a substantially dry tissue (i.e., substantially in the absence of plasma or like tissue fluids) to polymerize in less than 300 seconds at ambient temperature, for example, at approximately 21-25°C. Preferably, the initiator causes the monomer composition to polymerize in less than 150 seconds at ambient temperature, more preferably within 130 seconds. As used herein, a polymerization rate modifier is any material that changes the rate at which a polymerizable monomer would polymerize in the absence of that material. Preferably, the rate modifier accelerates the rate of the polymerization reaction.

In embodiments, the initiator or rate modifier is an accelerator or catalyst. In embodiments, the initiator and/or rate modifier is bioactive.

The applicator tip according to the present invention provides several advantages, including the ability to:
a) control the molecular weight distribution of the polymerized or cross-linked adhesive;
b) control the setting time of the polymerized or cross-linked adhesive;
c) provide precision and convenience in applying the adhesive to a tissue;
d) extend the shelf life of the monomer;
e) reduce the amount of unreacted monomer at the completion of the polymerization reaction, thus avoiding associated monomer odors after polymerization;
f) control the flow properties of applied cyanoacrylate adhesives;
g) provide a bioactive material to a wound site while simultaneously providing wound closure, protection, and/or coverage; and/or
h) any combination thereof.

According to embodiments of the present invention, a method is provided for applying at least one agent selected from the group consisting of polymerization initiators and polymerization rate modifiers to an applicator tip for adhesives, comprising:
dissolving or dispersing said agent in a low boiling point solvent to form a solution;
applying said solution to said applicator tip; and
drying said applicator tip;
wherein the low boiling point solvent comprises methanol.

In other embodiments, the present invention provides an applicator for a polymerizable adhesive, comprising:
a conduit for a fluid polymerizable adhesive material;
an applicator tip operably connected to said conduit so that fluid flowing through said conduit also flows through said applicator tip; and
at least one agent selected from the group consisting of polymerization initiators and polymerization rate modifiers on or in said applicator tip;
wherein said applicator tip has a gradient of said agent

### BRIEF DESCRIPTION OF THE DRAWINGS

Fig. 1 depicts an applicator of the invention, showing the distribution of a polymerization rate modifier or a polymerization initiator in an applicator tip applied with methanol.
Fig. 2 shows a cross section of the applicator of Fig. 1 along the A-A line.
Fig. 3 depicts an applicator showing the distribution of a polymerization rate modifier or a polymerization initiator on an applicator tip applied with acetone.
Fig. 4 shows a cross section of the applicator of Fig. 3 along the A-A line.
Fig. 5 shows the polymerization temperatures of a 2-octyl cyanoacrylate composition dispensed through applicator tips having an initiator applied with methanol and with acetone.
Fig. 6 shows the relationship between setting time (time required for polymerization) of a 2-octyl cyanoacrylate composition and concentration of initiator. The figure also shows the time required for polymerization of a 2-octyl cyanoacrylate composition dispensed through tips having an initiator applied with methanol and with acetone.

### DETAILED DESCRIPTION OF PREFERRED EMBODIMENTS

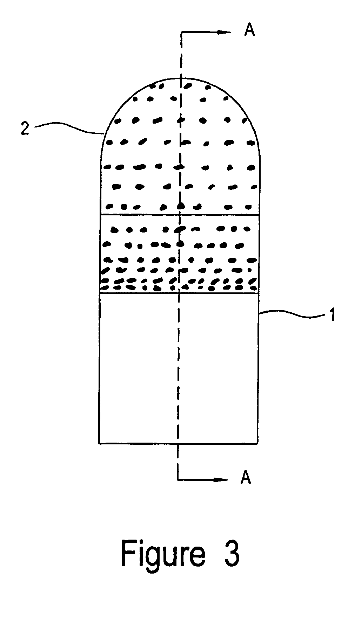

As mentioned above, PCT Application No. WO 96/40797, discloses the use of low boiling point solvents, such as acetone, ethanol, or mixtures thereof, to apply a polymerization or cross-linking initiator or rate modifier to an applicator tip. Cyanoacrylate adhesive compositions applied through such tips can show rapid polymerization, with concomitant production of heat. If the compositions are applied to living tissues, this might, in some instances, cause damage to the tissues and necrosis of underlying and/or adjacent living matter. Analysis of the tips produced by the methods of WO 96/40797 shows that the use of acetone to apply the initiator or rate modifier to the tip results in distribution of the material primarily on the outside, or near the exterior surface, of the tip as well as at the proximal, or bottom, portion. Such a distribution is shown in Figure 3.

To improve the polymerization characteristics of polymerizable monomer compositions delivered through applicator tips having a polymerization initiator and/or a polymerization rate modifier disposed thereon, the solvent used to apply the initiator or rate modifier was varied. It was discovered that the use of methanol to apply the initiator or rate modifier to an applicator tip provides unexpectedly superior polymerization characteristics to a polymerizable monomer composition in comparison to compositions applied through tips having an initiator and/or rate modifier applied using acetone. Polymerizable cyanoacrylate monomer compositions dispensed through such tips typically generate much less heat than the same compositions dispensed through tips prepared using acetone.

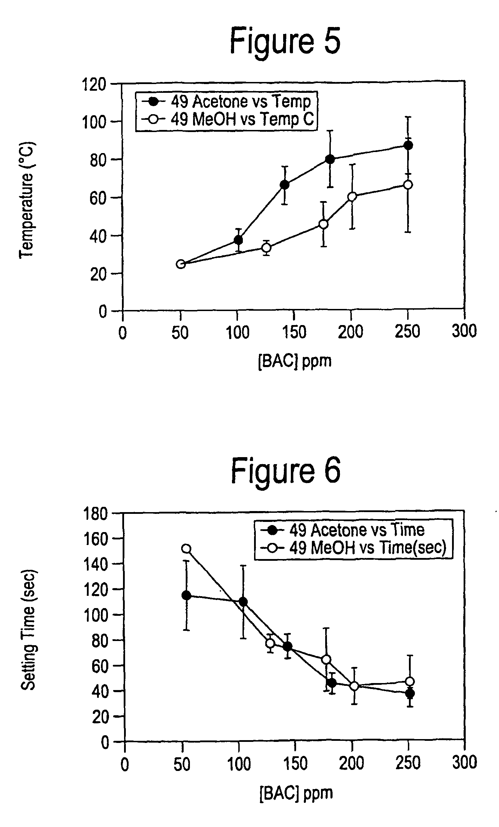

It can be shown, using thermal analysis techniques such as differential scanning calorimitry, that monomer compositions applied through such tips generate levels of heat that can be damaging to tissues. For example, as shown in Figure 5, a composition comprising 2-octyl cyanoacrylate dispensed through an applicator tip having an initiator (benzalkonium chloride) applied with acetone can generate enough heat at 200 seconds after initiation of polymerization to raise the temperature of the composition to approximately 80°C. However, the same composition dispensed through a tip having the same initiator applied using methanol shows a much lower level of heat generation (approximately 40°C).

Furthermore, as shown in Figure 6, the setting time, or the amount of time required for polymerization, of a 2-octyl cyanoacrylate composition is slightly longer when the cyanoacrylate composition is dispensed through a tip having a benzalkonium chloride initiator disposed thereon using methanol as compared to using acetone.

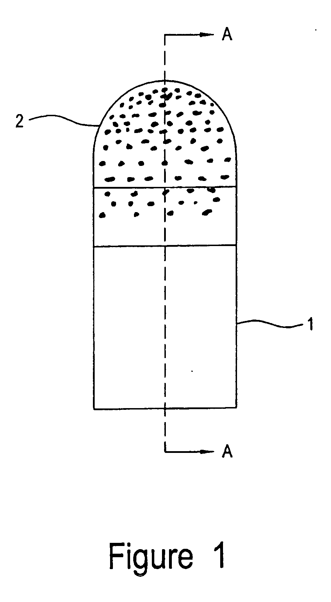

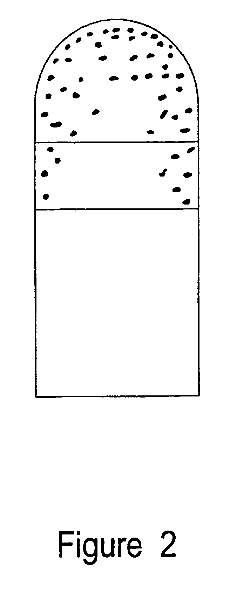

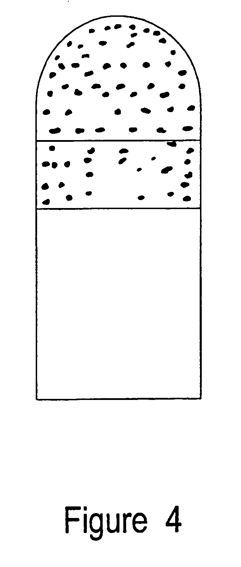

Figures 1-4 show effects of different solvents used to apply a polymerization initiator or a polymerization rate modifier to an applicator tip. The initiator or rate modifier is applied to the tip by pumping a liquid medium comprising the initiator or rate modifier through a syringe and onto the distal end of the tip. Figure 1 shows an applicator tip 2 of applicator 1 treated with a solution of an initiator or rate modifier dissolved in 110 µL of methanol, and subsequently dried for about 30 minutes. Figure 2 shows a cross-section of the applicator and tip in Figure 1. The initiator or rate modifier is present on the tip in a concentration gradient. The initiator or rate modifier concentration is highest at the top, or distal, end of the applicator tip and decreases towards the center and bottom, or proximal, end of the applicator tip. In contrast, Figure 3 shows an applicator tip treated with a solution of an initiator or rate modifier dissolved in 110 µL of acetone, and subsequently dried for about 30 minutes. Figure 4 shows a cross-section of the applicator and tip in Figure 3. The initiator or rate modifier is present on the tip in a concentration gradient that increases from the top (distal) end towards the bottom (proximal) end of the applicator tip.

The pattern of distribution of the material within the tip, when applied using a solvent comprising methanol, provides unexpectedly superior polymerization characteristics to monomer compositions dispensed through the tip as compared to compositions dispensed through tips prepared using other low boiling point solvents, such as acetone. The monomer compositions dispensed through tips prepared using solvents comprising methanol polymerize rapidly enough to make them convenient to apply; however, the polymerization that occurs does not result in the level of tissue damage often seen when other systems of cyanoacrylate delivery are used.

According to this aspect of the present invention, the solvent (also referred to herein as the liquid medium) for application of the material comprises methanol. Methanol can be used as the only solvent, or it can be present in mixtures of methanol with other low boiling point solvents, including low molecular weight ketones such as acetone. The mixture can be in any ratio. Preferably, the mixture is a ratio of methanol to other solvent of between 99:1 to 1:99. For example, the ratio of methanol to other solvent can be approximately 80:20 to 20:80, or 60:40 to 40:60. A ratio of at least 70:30, such as 80:20 or 90:10 or higher, is desirable in embodiments. Preferably, when the solvent comprises a component other than methanol, the other component is a low boiling point solvent having a vapor pressure of about 25 - 150 mm Hg at 20°C, such as about 30-125 mm Hg at 20°C, or about 40-100 mm Hg at 20°C, or mixtures thereof. In embodiments, low molecular weight solvents can be used. Included are low molecular weight ketones and alcohols.

The material may be applied to the applicator tip by spraying, dipping, injecting, or brushing the applicator tip with the liquid medium containing the material. It is preferably applied to the tip by dipping or injecting. For example, it may be applied to the tip by pumping of the liquid medium, for example, through a syringe, onto the distal end of the tip.

In exemplary embodiments, preparing an applicator for dispensing polymerizable monomeric compositions includes applying a material to a porous polyethylene tip, which is attached to a butyrate applicator tube. Use of solvents comprising methanol also provides adequate bonding of the butyrate applicator tube to a polyethylene applicator tip. The solvent used to apply the material to the tip also helps bond the polyethylene applicator tip to the butyrate applicator tube. When using acetone, damage to the tube and/or tip can occur if too much acetone is used. Solvents comprising methanol, while still providing the bonding that is necessary to hold the tip to the tube, allow the use of a greater range of solvent amounts to apply the material to the tip.

An anisotropic distribution or a concentration gradient of material in the applicator tip can be obtained with the use of a methanol-containing solvent. The distribution of the material may be varied depending on the solvent or solvents used to apply it and on the wetting characteristics of the solvent and tip. In general, the wetting characteristics of the solvent should be such that the surface tension is close enough to that of the tip material to wet at least the surface of the tip.

The material applied to the applicator tip can be any material, but is preferably an initiator that initiates polymerization and/or cross-linking of the monomer; or a polymerization rate modifier, which modifies the rate of polymerization of the monomer. The material may be applied to a surface portion or to the entire surface of the applicator tip. Preferably, only a portion of the exterior of the applicator tip is treated with the material.

Particular initiators and rate modifiers for particular monomers may be readily selected by one of skill in the art without undue experimentation. Control of the molecular weight distribution of the applied adhesive can be enhanced by selection of the concentration and functionality of the initiator or rate modifier vis-a-vis the selected monomer. Suitable polymerization initiators and rate modifiers for cyanoacrylate compositions include, but are not limited to, detergent compositions; surfactants, including nonionic surfactants such as polysorbate 20 (e.g., Tween 20™; ICI Americas), polysorbate 80 (e.g., Tween 80™; ICI Americas), and poloxamers; cationic surfactants such as tetrabutylammonium bromide; anionic surfactants, including quaternary ammonium halides such as benzalkonium chloride or its pure components, and benzethonium chloride; stannous octoate (tin (II) 2-ethylhexanoate), and sodium tetradecyl sulfate; and amphoteric or zwitterionic surfactants such as dodecyldimethyl(3-sulfopropyl) ammonium hydroxide, inner salt; amines, imines, and amides, such as imidazole, tryptamine, urea, arginine and povidine; phosphines, phosphites and phosphonium salts, such as triphenylphosphine and triethyl phosphite; alcohols such as ethylene glycol; methyl gallate; ascorbic acid; tannins and tannic acid; inorganic bases and salts, such as sodium bisulfite, magnesium hydroxide, calcium sulfate and sodium silicate; sulfur compounds such as thiourea and polysulfides; polymeric cyclic ethers such as monensin, nonactin, crown ethers, calixarenes and polymeric epoxides; cyclic and acyclic carbonates, such as diethyl carbonate; phase transfer catalysts such as Aliquat™ 336 (General Mills, Inc., Minneapolis, MN); organometallics; manganese acetylacetonate; radical initiators and radicals, such as di-t-butyl peroxide and azobisisobutyronitrile; and bioactive compounds or agents.

In preferred embodiments, the initiator may be a bioactive material, including quaternary ammonium halides such as alkylbenzyldimethylammonium chloride (benzalkonium chloride; BAC) its pure components, or mixtures thereof, especially those with an alkyl containing 6-18 carbon atoms; benzethonium chloride; and salts of sulfadiazine. Cobalt napthenate can be used as an accelerator for peroxide.

The polymerizable and/or cross-linkable material may also contain an initiator and/or a rate modifier which is inactive until activated by a catalyst or accelerator (included within the scope of the term "initiator" as used herein) in the applicator tip. Initiators activated by stimulation such as heat and/or light (e.g., ultraviolet or visible light) are also suitable if the tip and/or applicator is appropriately subjected to such stimulation.

The initiator or rate modifier is dissolved or otherwise dispersed in the solvent and applied to the applicator tip in any effective amount. An effective amount is that amount of initiator or rate modifier that effects polymerization to a gel point on dry tissue in less than 300 seconds, preferably within 150 seconds, and more preferably within 130 seconds, at ambient temperature, such as approximately 21-25°C. The coated applicator is then allowed to dry, thereby evaporating the solvent. In embodiments, the applicator is allowed to dry for about 5 to 35 minutes. In embodiments, the amount of initiator or modifier dissolved or dispersed in the solvent may be about, or less than, 25 wt.%, preferably less than 10 wt.% and more preferably less than 1 wt.%. The amount of initiator or rate modifier dissolved or dispersed in the solvent may be any effective amount. In the case where a quaternary ammonium halide is the initiator or rate modifier, the effective amount is preferably between 100 and 250 ppm or more in 110 µl. In the case of salts of sulfadiazine, the effective amount is preferably approximately 50 ppm or more in 110 µl. The effective amount for each initiator and adhesive monomer combination can easily be determined by one of ordinary skill in the art.

To determine the polymerization time, an appropriate volume of a solution of the initiator prepared in a volatile solvent is placed in a differential scanning calorimetric pan. The volatile solvent is allowed to dry under ambient conditions. Alternatively, the appropriate quantity of the initiator is dispensed directly onto the differential scanning calorimetric pan. In either of the abovementioned cases, 25 µl of the chosen monomer solution is pipetted into the pan. The time taken for the monomer composition to polymerize to the point of a gel is the polymerization time.

In embodiments, the initiator and/or the rate modifier can be, but does not have to be, bioactive. In embodiments where the initiator and/or the rate modifier is bioactive, the method of the invention can be used to close, cover, or protect tissue and wounds while simultaneously providing a bioactive material to the tissue or wound.

In embodiments where the initiator is also a bioactive material, the bioactive material is applied onto the tip in an amount that is effective to initiate polymerization and to be effective for the biological activity intended (e.g., in a sufficient amount to be antiseptic). The bioactive material is selected in conjunction with the polymerizable monomer to be dispensed such that the bioactive material functions as an initiator or rate modifier for the monomer. During dispensing of the monomer composition, the bioactive material is mixed with the monomer composition. In embodiments, the bioactive material can be released to the tissue to be treated at a copstant, or near constant, rate over a period of time while the polymerized composition is in contact with the wound site.

The present invention is also directed to a method of applying the adhesive composition utilizing an applicator comprising a tip having a polymerization initiator and/or a polymerization rate modifier, applied thereon. According to the invention, any appropriate design for the applicator can be used. Such applicator designs include, but are not limited to, crushable swab applicators, syringes, adhesive guns, pipettes, eyedroppers, vials, and the like with various dispensing nozzles or tips.

For example, the applicator tip may be detachable from the applicator container holding the polymerizable and/or cross-linkable material. Such an applicator tip could be attached to the applicator container prior to use and detached from the applicator container subsequent to use in order to prevent premature polymerization or cross-linking of the unapplied material in the applicator container. At this point the applicator tip may be discarded and a new applicator tip may be attached to the applicator container for subsequent use, or the applicator tip may be cleaned and reused.

Additionally, the applicator tip according to the present invention may comprise multiple parts, with at least one part having the initiator and/or rate modifier.

For example, the component containing the initiator and/or rate modifier, may be fabricated separately from the other component(s) of the applicator tip and assembled prior to attachment to the applicator container.

The applicator tip may also be in the form of a nozzle for atomizing liquid polymerizable and/or cross-linkable materials. Conical, flat spray or condensed stream nozzles are suitable.

The applicator tip and the applicator container may be an integral or even monolithic unit. The unit may be preformed as a single piece and charged with polymerizable and/or cross-linkable material. After application of material from the applicator container, the unit may be discarded. Additionally, such an integral or monolithic applicator tip/applicator container unit may be fashioned to provide the capability of recharging the unit with new material as a multiple use device.

The applicator tip may be composed of any of a variety of materials including polymerized materials such as plastics, foams, rubber, thermosets, films, or membranes. In embodiments, the applicator tip may be made from polyesters, polyolefins such as polyethylene, or polyamides. In embodiments, the applicator may be made from polyethylene, such as that sold by Porex Technologies Corp. (Fairburn, GA) under the name LabPor®. Additionally, the applicator tip may be composed of materials such as metal, glass, paper, ceramics, and the like. The applicator tip material may be porous, absorbent, or adsorbent in nature to enhance and facilitate loading of the initiator and/or the rate modifier on or within the applicator tip. For example, the applicator tip may be composed of a material having random pores, capillaries, a honeycomb material, a material having a woven pattern, etc. The degree of porosity will depend on the materials being used, and can be determined easily by one of ordinary skill in the art. Porosity is the open volume within the pores of an applicator tip divided by the total volume of the applicator tip.

In embodiments, the applicator tip may be porous and have an average pore size of about 1 µm to about 500 µm. Generally, according to the present invention, an applicator tip having an average pore size of about 20 µm is used with a polymerizable material having a viscosity of about 1-30 cPs, preferably about 2-18 cPs, and more preferably 7 cPs at 25°C. When the polymerizable and/or cross-linkable material has a viscosity higher than 7 cPs, the average pore size of the applicator tip is generally increased. For example, an applicator tip having an average pore size of about 140 µm is preferably used with a polymerizable material having a viscosity of about 30-500 cPs, preferably about 35-350 cPs, and more preferably about 250 cPs at 25°C. In embodiments, an applicator tip has a porosity of less than or equal to 80 percent.

In embodiments, when using a porous applicator, the amount of initiator or rate modifier necessary to initiate and/or to modify the rate of polymerization and/or cross-linking increases as the pore size of the applicator tip increases.

The applicator tip according to the present invention, where it connects to the applicator tube, may have an elongated tubular portion, out of which the mixed polymerizing and/or cross-linking material is expelled. A portion of the applicator tip which is immediately downstream of the applicator tube is advantageously porous in order to avoid a sharp pressure drop and ensure a constant mixed ratio profile. The structure can preferably trap fragments of any barriers or materials used to separate one or more components within the applicator container so that they will not clog the device or contact the patient.

When using a porous applicator tip to apply the adhesive composition, the composition preferably is not expressed directly through the applicator tip in a continuous motion. According to embodiments of the present invention, the adhesive composition is (1) expressed to the end or part way to the end of the applicator tip, (2) the pressure is released to draw the composition back into the applicator, and (3) the composition is then subsequently expressed through the applicator tip in a continuous motion. This is called a suck-back method of applying the adhesive composition of the present invention. As a result, the adhesive composition polymerizes more slowly than if it had been expressed directly through the tip.

The initiator, and/or rate modifier, may be in the form of a solid, such as a powder or a solid film, or in the form of a liquid, such as a viscous or paste-like material. The tip may also include a variety of additives, such as surfactants or emulsifiers. Preferably, the initiator and/or rate modifier, is soluble or otherwise dispersible in the polymerizable and/or cross-linkable material, and/or comprises or is accompanied by at least one surfactant which helps it co-elute with the polymerizable and/or cross-linkable material. In embodiments, the surfactant may help solubilize it in the polymerizable and/or cross-linkable material. The initiator and/or rate modifier, agent thus mixes with the adhesive composition as the mixture passes through the tip.

## Claims

1. A method of applying at least one agent selected from the group consisting of polymerization initiators and polymerization rate modifiers to an applicator tip for adhesives, comprising:
dissolving or dispersing said agent in a low boiling point solvent to form a solution;
applying said solution to said applicator tip; and
drying said applicator tip;
wherein the low boiling point solvent comprises methanol.

2. The method of claim 1, wherein the agent is dissolved in the low boiling point solvent.

3. The method of claim 1 or 2, wherein the agent is selected from the group consisting of polysorbate 20, polysorbate 80, poloxamers, tetrabutylammonium bromide, alkylbenzylalkonium chloride, stannous octoate (tin (II) 2-ethylhexanoate), sodium tetradecyl sulfate, and dodecyldimethyl(3-sulfopropyl)ammonium hydroxide.

4. The method of claim 1 or 2, wherein the agent is selected from the group consisting of imidazole, tryptamine, urea, arginine, povidine, triphenylphosphine, triethyl phosphite, ethylene glycol, methyl gallate, ascorbic acid, tannins, tannic acid, sodium bisulfite, magnesium hydroxide, calcium sulfate, sodium silicate, thiourea, monensin, nonactin, crown ethers, calixarenes, polymeric epoxides, diethyl carbonate, di-t-butyl peroxide, and azobisisobutyronitrile.

5. The method of claim 1 or 2, wherein the agent is alkylbenzyldimethylammonium chloride with an alkyl containing 6-18 carbon atoms, its pure components, or mixtures thereof.

6. The method of any one of claims 1-5, wherein the agent comprises at least one compound that is both (i) at least one member selected from the group consisting of polymerization initiators and polymerization rate modifiers and (ii) a bioactive material.

7. The method of any one of claims 1-6, wherein said solvent further comprises a low boiling point ketone or alcohol other than methanol.

8. The method of any one of claims 1-7, wherein said solvent further comprises acetone.

9. The method of any one of claims 1-8, comprising applying said solution to a distal end of the applicator tip and forming a concentration gradient of said agent that decreases from said distal end of the applicator tip towards a center and a proximal end of the applicator tip.

10. The method of any one of claims 1-9, comprising affixing said applicator tip to an applicator tube before applying said solution to said applicator tip.

11. The method of any one of claims 1-9, comprising placing said applicator tip on or in an applicator tube after applying said solution to said applicator tip.

12. The method of any one of claims 1-11, wherein said applicator tip comprises a porous polyolefin, polyester, or polyamide.

13. The method of any one of claims 1-12, wherein said applicator tip comprises porous polyethylene.

14. The method of any one of claims 1-13, wherein said applicator tip has an average pore size of about 1 µm to about 500 µm.

15. An applicator tip having at least one agent applied thereto made by the method of any one of claims 1-14.

16. An applicator for a polymerizable adhesive, comprising:
a conduit for a fluid polymerizable adhesive material;
an applicator tip operably connected to said conduit so that fluid flowing through said conduit also flows through said applicator tip; and
at least one agent selected from the group consisting of polymerization initiators and polymerization rate modifiers on or in said applicator tip;
wherein said applicator tip has a gradient of said agent;
wherein the gradient shows a decrease in concentration of the agent from a distal end to a proximal end of the applicator tip.

17. The applicator of claim 16, wherein the agent is a bioactive material and is also at least one member selected from the group consisting of polymerization initiators and polymerization rate modifiers.

18. The applicator of any one of claims 16-17, further comprising a container of polymerizable adhesive physically separated from said applicator tip.

19. The applicator of any one of claims 16-18, wherein said polymerizable adhesive comprises a 1,1-disubstituted ethylene monomer.

20. The applicator of claim 19, wherein said monomer is a cyanoacrylate.

21. An applicator for a polymerizable adhesive, comprising:
a conduit for a fluid polymerizable adhesive material; and
an applicator tip according to claim 15.

22. The applicator of any one of claims 16-21, wherein said applicator contains said polymerizable adhesive material.

23. The applicator of any one of claims 16-22, wherein the polymerizable adhesive composition, upon passing through the applicator tip, solubilizes or disperses and mixes with said agent, thus producing a medical adhesive composition.

24. The applicator of any one of claims 16-23, wherein the applicator is sterilized.

## Patentansprüche

1. Verfahren zur Anwendung zumindest eines Mittels, ausgewählt aus der Gruppe bestehend aus Polymerisationsinitiatoren und Polymerisationsratenmodifikatoren an einer Applikatorspitze für Klebstoffe, das Folgendes aufweist, nämlich
Auflösen oder Verteilen des Mittels in einem Lösungsmittel niedrigen Siedepunktes, um eine Lösung zu bilden;
Aufbringen der Lösung an die Applikatorspitze; und
Trocknen der Applikatorspitze;
wobei das Lösungsmittel niedrigen Siedepunktes Methanol aufweist.

2. verfahren nach Anspruch 1, wobei das Mittel in dem Lösungsmittel niedrigen Siedepunktes aufgelöst ist.

3. Verfahren nach Anspruch 1 oder 2, wobei das Mittel ausgewählt ist aus der Gruppe bestehend aus Polysorbat 20, Polysorbat 80, Poloxameren, Tetrabutylammoniumbromid, Alkylbenzylalkoniumchlorid, Zinnoktoat (Zinn-(II)-2-Ethylhexanoat), Natriumtetradecylsulfat und Dodecyldimethyl(3-sulfopropyl)-ammoniumhydroxid.

4. Verfahren nach Anspruch 1 oder 2, wobei das Mittel ausgewählt ist aus der Gruppe bestehend aus Imidazol, Typtamin, Harnstoff, Arginin, Povidin, Triphenylphosphin, Triethylphosphit, Ethylenglycol, Methylgallat, Ascorbinsäure, Tannine, Tanninsäure, Natriumbisulfit, Magnesiumhydroxid, Calciumsulfat, Natriumsilicat, Thioharnstoff, Monensin, Nonactin, Kronenethern, Kalixarenen, polymeren Epoxiden, Diethylcarbonat, Dit-Butylperoxid und Azobisisobutyronitril.

5. Verfahren nach Anspruch 1 oder 2, wobei das Mittel ein Alkylbenzyldimethylammoniumchlorid mit einem Alkyl ist, das 6 - 18 Kohlenstoffatome enthält, dessen reine Zusammensetzungen bzw. Gemische davon.

6. Verfahren nach einem der Ansprüche 1 bis 5, wobei das Mittel zumindest eine Verbindung aufweist, die beides ist, (i) zumindest ein Mitglied ausgewählt aus der Gruppe bestehend aus den Polymerisationsinitiatoren und den Polymerisationsratenmodifikatoren und (ii) ein bioaktives Material.

7. Verfahren nach einem der Ansprüche 1 bis 6, wobei das Lösungsmittel ferner Folgendes aufweist, nämlich ein Keton niedrigen Siedepunktes oder einen Alkohol unterschiedlich zu Methanol.

8. Verfahren nach einem der Ansprüche 1 bis 7, wobei das Lösungsmittel ferner Aceton aufweist.

9. Verfahren nach einem der Ansprüche 1 bis 8, das Folgendes aufweist, nämlich Aufbringen der Lösung an einem distalen Ende der Applikatorspitze und Ausbilden eines Konzentrationsgradienten des Mittels, der von dem distalen Ende der Applikatorspitze hin zu einem Zentrum und einem proximalen Ende der Applikatorspitze abnimmt.

10. Verfahren nach einem der Ansprüche 1 bis 9, das Folgendes aufweist, nämlich Befestigen der Applikatorspitze an einer Applikatortube, und zwar vor Aufbringen der Lösung an die Applikatorspitze.

11. Verfahren nach einem der Ansprüche 1 bis 9, das Folgendes aufweist, nämlich ein Platzieren der Applikatorspitze an oder in einer Applikatortube nach dem Aufbringen der Lösung an der Applikatorspitze.

12. Verfahren nach einem der Ansprüche 1 bis 11, wobei die Applikatorspitze ein poröses Polyolefin, ein Polyester oder ein Polyamid aufweist.

13. Verfahren nach einem der Ansprüche 1 bis 12, wobei die Applikatorspitze ein poröses Polyethylen aufweist.

14. Verfahren nach einem der Ansprüche 1 bis 13, wobei die Applikatorspitze eine durchschnittliche Porengröße von etwa 1 µm bis etwa 500 µm aufweist.

15. Applikatorspitze, an der zumindest ein Mittel angebracht ist, hergestellt durch das Verfahren nach einem der Ansprüche 1 bis 14.

16. Applikator für einen polymerisierbaren Klebstoff, der Folgendes aufweist, nämlich
eine Leitung für ein fluides polymerisierbares Klebstoffmaterial;
eine Applikatorspitze, die bedienbar mit der Leitung verbunden ist, so dass das durch die Leitung fließende Fluid auch durch die Applikatorspitze fließt; und
zumindest ein Mittel, das ausgewählt ist aus der Gruppe bestehend aus den Polymerisationsinitiatoren und den Polymerisationsratenmodifikatoren an oder in der Applikatorpitze;
wobei die Applikatorspitze einen Gradienten des Mittels aufweist;
wobei der Gradient eine Abnahme in der Konzentration des Mittels von einem distalen Ende zu einem proximalen Ende der Applikatorspitze aufweist.

17. Applikator nach Anspruch 16, wobei das Mittel ein bioaktives Material ist und auch zumindest ein Mitglied ist, ausgewählt aus der Gruppe bestehend aus den Polymerisationsinitiatoren und den Polymerisationsratenmodifikatoren.

18. Applikator nach einem der Ansprüche 16 bis 17, der weiterhin einen Behälter mit polymerisierbarem Klebstoff aufweist, der physikalisch getrennt von der Applikatorspitze ist.

19. Applikator nach einem der Ansprüche 16 bis 16, wobei der polymerisierbare Klebstoff ein 1,1-disubstituiertes Ethylenmonomer aufweist.

20. Applikator nach Anspruch 19, wobei das Monomer ein Cyanoacrylat ist.

21. Applikator für einen polymerisierbaren Klebstoff, der Folgendes aufweist nämlich
eine Leitung für ein fluides polymerisierbares Klebstoffmaterial; und
eine Applikatorspitze gemäß Anspruch 15.

22. Applikator nach einem der Ansprüche 16 bis 21, wobei der Applikator das polymerisierbare Klebstoffmaterial aufweist.

23. Applikator nach einem der Ansprüche 16 bis 22, wobei sich die polymerisierbare Klebstoffzusammensetzung, durch die Applikatorspitze laufend, mit dem Mittel solubilisiert bzw. mit dem Mittel verteilt und mischt, und somit eine medizinische Klebstoffzusammensetzung erzeugt.

24. Applikator nach einem der Ansprüche 16 bis 23, wobei der Applikator sterilisiert ist.

## Revendications

1. Procédé d'application d'au moins un agent choisi dans le groupe constitué par les initiateurs de polymérisation et les modificateurs de vitesse de polymérisation, sur un embout d'applicateur d'adhésifs, comprenant les étapes consistant à :
dissoudre ou disperser ledit agent dans un solvant à bas point d'ébullition pour former une solution ;
appliquer ladite solution sur ledit embout d'applicateur ; et
sécher ledit embout d'applicateur ;
dans lequel le solvant à bas point d'ébullition comprend le méthanol.

2. Procédé selon la revendication 1, dans lequel l'agent est dissout dans le solvant à bas point d'ébullition.

3. Procédé selon la revendication 1 ou 2, dans lequel l'agent est choisi dans le groupe comprenant le polysorbate 20, le polysorbate 80, des poloxamères, le bromure de tétrabutylammonium, le chlorure d'alkylbenzylalkonium, l'octoate stanneux (étain(II) 2-éthylhexanoate), le tétradécylsulfate de sodium et l'hydroxyde de dodécyldiméthyl(3-sulfopropyl)ammonium.

4. Procédé selon la revendication 1 ou 2, dans lequel l'agent est choisi dans le groupe comprenant l'imidazole, la tryptamine, l'urée, l'arginine, la povidine, la triphénylphosphine, le triéthyle phosphite, l'éthylène-glycol, le méthyle gallate, l'acide ascorbique, les tannins, l'acide tannique, le bisulfite de sodium, l'hydroxyde de magnésium, le sulfate de calcium, le silicate de sodium, la thiourée, la monensine, la nonactine, les "crown" éthers, les calixarènes, des polymères époxydes, le diéthyle carbonate, le di-t-butyle peroxyde, et l'azobisisobutyronitrile.

5. Procédé selon la revendication 1 ou 2, dans lequel l'agent est le chlorure l'alkybenzyldiméthylammonium avec un alkyle contenant 6 à 18 atomes de carbone, ses composants purs, ou leurs mélanges.

6. Procédé selon l'une quelconque des revendications 1 à 5, dans lequel l'agent comprend au moins un composé qui est à la fois (i) au moins un élément choisi dans le groupe comprenant les initiateurs de polymérisation et les modificateurs de vitesse de polymérisation et (ii) un matériau bioactif.

7. Procédé selon l'une quelconque des revendications 1 à 6, dans lequel ledit solvant comprend en outre une cétone à bas point d'ébullition ou un alcool autre que le méthanol.

8. Procédé selon l'une quelconque des revendications 1 à 7, dans lequel ledit solvant comprend en outre l'acétone.

9. Procédé selon l'une quelconque des revendications 1 à 8, comprenant les étapes consistant à appliquer ladite solution à une extrémité distale de l'embout d'applicateur, et à former un gradient de concentration dudit agent qui diminue depuis ladite extrémité distale de l'embout d'applicateur vers un centre et une extrémité proximale de l'embout d'applicateur.

10. Procédé selon l'une quelconque des revendications 1 à 9, comprenant les étapes consistant à fixer ledit embout d'applicateur à un tube d'applicateur, avant d'appliquer ladite solution au dit embout d'applicateur.

11. Procédé selon l'une quelconque des revendications 1 à 9, comprenant l'étape consistant à placer ledit embout d'applicateur sur ou dans un tube d'applicateur après application de ladite solution au dit embout d'applicateur.

12. Procédé selon l'une quelconque des revendications 1 à 11, dans lequel ledit embout d'applicateur inclut une polyoléfine, un polyester ou un polyamide poreux.

13. Procédé selon l'une quelconque des revendications 1 à 12, dans lequel ledit embout d'applicateur comprend du polyéthylène poreux.

14. Procédé selon l'une quelconque des revendications 1 à 13, dans lequel ledit embout d'applicateur a une taille de pores moyenne d'environ 1 µm à environ 500 µm.

15. Embout d'applicateur ayant au moins un agent appliqué dessus, fabriqué par le procédé selon l'une quelconque des revendications 1 à 14.

16. Applicateur d'adhésif pouvant être polymérisé, comprenant :
un conduit pour le matériau adhésif fluide pouvant être polymérisé ;
un embout d'applicateur raccordé de manière fonctionnelle au dit conduit, de sorte que le fluide s'écoulant dans ledit conduit s'écoule aussi dans ledit embout d'applicateur ; et
au moins un agent choisi dans le groupe comprenant des initiateurs de polymérisation et des modificateurs de vitesse de polymérisation sur ou dans ledit embout d'applicateur ;
dans lequel ledit embout d'applicateur présente un gradient dudit agent ;
dans lequel le gradient présente une diminution de concentration de l'agent depuis une extrémité distale vers une extrémité proximale de l'embout d'applicateur.

17. Applicateur selon la revendication 16, dans lequel l'agent est un matériau bioactif et est aussi au moins un élément choisi dans le groupe constitué par les initiateurs de polymérisation et les modificateurs de vitesse de polymérisation.

18. Applicateur selon l'une quelconque des revendications 16 et 17, comprenant en outre un récipient d'adhésif pouvant être polymérisé, séparé physiquement dudit embout d'applicateur.

19. Applicateur selon l'une quelconque des revendications 16 à 18, dans lequel ledit adhésif pouvant être polymérisé comprend un monomère éthylène 1,1-disubstitué.

20. Applicateur selon la revendication 19, dans lequel ledit monomère est un cyanoacrylate.

21. Applicateur pour un adhésif pouvant être polymérisé, comprenant :
un conduit pour un matériau adhésif fluide pouvant être polymérisé ; et
un embout d'applicateur selon la revendication 15.

22. Applicateur selon l'une quelconque des revendications 16 à 21, dans lequel ledit applicateur contient ledit matériau adhésif pouvant être polymérisé.

23. Applicateur selon l'une quelconque des revendications 16 à 22, dans lequel la composition adhésive pouvant être polymérisée, lorsqu'elle traverse l'embout d'applicateur, se solubilise ou se met en dispersion et se mélange avec ledit agent, produisant ainsi une composition adhésive médicale.

24. Applicateur selon l'une quelconque des revendications 16 à 23, dans lequel l'applicateur est stérilisé.
